# FASCICULE DE BREVET EUROPEEN

(11) **EP 1 694 338 B1**
(45) Date de publication et mention de la délivrance du brevet: **15.10.2008**
(21) Numéro de dépôt: 04816432.1
(22) Date de dépôt: 17.12.2004
(51) Int. Cl.: A61K 31/675, A61K 31/555, A61K 31/337, A61K 31/513, A61K 45/06, A61K 31/00, A61P 35/00

(54) **UTILISATION DE MIMETIQUES DE LA SUPEROXYDE DISMUTASE ET DE LA GLUTATHION REDUCTASE COMME AGENTS ANTI-CANCEREUX**
VERWENDUNG VON SUPEROXIDDISMUTASE- UND REDUCTASE-GLUTATHION-MIMETIKA IN FORM VON ANTI-KREBSMITTELN
USE OF MIMETICS OF SUPEROXIDE DISMUTASE AND OF REDUCTASE GLUTATHIONE IN THE FORM OF ANTICANCER DRUGS

(30) Priorité: 18.12.2003 FR 0314933
(43) Date de publication de la demande: 30.08.2006
(73) Titulaire: UNIVERSITE RENE DESCARTES, (PARIS V), F-75006 Paris (FR); Protexel, 75014 Paris (FR)
(72) Inventeur: WEILL, Bernard, F-95600 EAUBONNE (FR); BATTEUX, Frédéric, F-75015 PARIS (FR); LAURENT, Alexis, F-94410 SAINT-MAURICE (FR)
(74) Mandataire: Vialle-Presles, Marie José
(86) Numéro de dépôt international: PCT/FR2004/003298
(87) Numéro de publication internationale: WO 2005/060976

(56) Documents cités:
- WO-A-01/19823
- WO-A-84/04922
- WO-A-97/49390
- WO-A-02/060383
- WO-A-02/087579
- WO-A-03/099296
- FR-A- 2 766 712
- US-B1- 6 391 895
- BEDDA SASSIA ET AL: "Mangafodipir prevents liver injury induced by acetaminophen in the mouse." JOURNAL OF HEPATOLOGY. NOV 2003, vol. 39, no. 5, novembre 2003 (2003-11), pages 765-772, XP001197347 ISSN: 0168-8278
- LAURENT A ET AL: "Controlling tumor growth by modulating endogenous production of reactive oxygen species" CANCER RESEARCH 01 FEB 2005 UNITED STATES, vol. 65, no. 3, 1 février 2005 (2005-02-01), pages 948-956, XP002332740 ISSN: 0008-5472

## Description

La présente invention est relative à l'utilisation d'un mimétique chimique de la superoxyde dismutase (SOD), notamment le Mangafodipir, pour inhiber la croissance tumorale, et potentialiser les effets de traitements antitumoraux sur les cellules tumorales tout en inhibant leurs effets toxiques sur les cellules normales.

Le terme : « formes réactives de l'oxygène » (FRO) englobe un ensemble de dérivés réduits de l'oxygène, comme l'anion superoxyde (O₂^{•-}), le peroxyde d'hydrogène (H₂O₂), ou le radical hydroxyl (OH^{•}). Ces dérivés sont normalement générés par le métabolisme cellulaire, en particulier dans les mitochondries, lors de la réduction de l'oxygène moléculaire en H₂O. Ils sont en outre produits en quantités importantes dans certaines conditions, par exemple lors de l'exposition aux rayonnements ionisants ou aux rayons ultraviolets, ou de l'exposition à certains produits chimiques.

Les formes réactives de l'oxygène étant très toxiques, les cellules disposent de différents moyens de les neutraliser. Parmi ces moyens de détoxification figurent en particulier des enzymes « anti-oxydantes » parmi lesquelles on citera les superoxyde dismutases (SOD ; EC 1.15.1.1) qui catalysent la dismutation de l'anion superoxyde en peroxyde d'hydrogène + O₂, et les enzymes intervenant ensuite dans la détoxification du peroxyde d'hydrogène, telles que la catalase (EC 1.11.1.6) qui catalyse la dismutation du peroxyde d'hydrogène (2 H₂O₂ → O₂ + 2 H₂O), la glutathion-peroxydase (EC 1.11.1.9) qui catalyse la réduction du peroxyde d'hydrogène par le glutathion réduit (GSH), en produisant du glutathion oxydé (GSSG) et de l'eau (2 GSH + H₂O₂ → GSSG + 2 H₂O), et la glutathion-réductase (EC 1.8.1.7), qui régénère le GSH selon la réaction GSSG + NADPH + H⁴ → 2 GSH + NADP⁴.

Lorsque la production de formes réactives de l'oxygène excède les capacités de détoxification de la cellule, les effets toxiques de ces dérivés se manifestent, et peuvent induire des dommages importants au niveau de constituants cellulaires tels que les protéines, les lipides membranaires, ou l'ADN. Le stress oxydant ainsi généré joue un rôle majeur dans l'apparition et le développement de diverses maladies, notamment des pathologies inflammatoires et auto-immunes, et des cancers.

Il est à l'heure actuelle généralement admis que les formes réactives de l'oxygène interviennent dans la pathogenèse de nombreux cancers. Toutefois, il apparaît que leurs effets mettent en jeu des mécanismes complexes, qui sont loin d'être élucidés.

En quantités sub-létales, les FRO peuvent favoriser l'apparition de cancers, par exemple en provoquant des mutations au niveau des régions codantes ou des régions régulatrices, ou en inhibant, ou au contraire en stimulant l'expression de gènes impliqués dans la régulation de la prolifération ou de la différenciation cellulaires, ou de l'apoptose. Il a ainsi été proposé d'utiliser des antioxydants dans le cadre de traitements curatifs ou préventifs de différents cancers. Par exemple, une alimentation supplémentée en antioxydants, notamment en vitamine E, a été préconisée dans le but de prévenir le cancer.

A fortes concentrations, les FRO peuvent induire directement la mort cellulaire, notamment en provoquant des réactions de peroxydation lipidique et protéique, qui peuvent favoriser la dépolarisation mitochondriale et ainsi accélérer les phases effectrices de l'apoptose. Cette activation de l'apoptose par les FRO peut constituer un moyen de détruire les cellules tumorales.

Par exemple, les traitements par radiothérapie reposent essentiellement sur l'induction d'une surproduction de FRO dans les cellules tumorales. De même, de nombreuses molécules utilisées dans la chimiothérapie des cancers induisent dans les cellules une surproduction de FRO, qui serait responsable, au moins en partie, de l'effet anti-tumoral de ces molécules.

Les molécules anticancéreuses pouvant induire une production de FRO peuvent appartenir à différentes classes thérapeutiques. On citera notamment des agents intercalants, par exemple des anthracyclines comme la doxorubicine qui inhibe la réplication et induit des lésions de l'ADN ; des inhibiteurs de la topoisomérase-2 comme l'étoposide qui induit des cassures de l'ADN ; des anti-métabolites comme le 5-fluoro-uracile ; des agents électrophiles comme la mitomycine C et des dérivés du platine [cisplatine (YOKOMIZO et al., Cancer Res, 55: 4293-4296 1995), et oxaliplatine] ; des poisons du fuseau comme les taxanes ; et des anti-récepteurs hormonaux comme le tamoxifène (FERLINI et al., Br J Cancer, 79, 257-263, 1999).

Toutefois, l'une des principales limitations à l'utilisation de ces molécules anticancéreuses découle du fait que leur action peut aussi entraîner la mort de cellules normales et conduire à des lésions, parfois irréversibles, aux conséquences très préjudiciables.

La plupart des molécules anticancéreuses détruisent préférentiellement les cellules se divisant rapidement. Leur toxicité vis-à-vis des cellules saines est donc généralement moindre que vis-à-vis des cellules tumorales. Cependant, il existe dans certains tissus des cellules dont le taux de division est très rapide, et qui sont donc particulièrement sensibles aux effets toxiques des anti-cancéreux. Il s'agit notamment des cellules hématopoïétiques en voie de différenciation de la moelle osseuse. La myélotoxicité constitue la plus fréquente des toxicités associées à la chimiothérapie et est associée à la majeure partie des traitements antitumoraux. Elle touche essentiellement les leucocytes et les plaquettes, et se traduit notamment par une leucopénie qui augmente le risque infectieux chez les patients traités.

Certaines molécules anticancéreuses présentent en outre une cytotoxicité visant plus spécifiquement certains tissus ou organes. A titre d'exemples : les anthracyclines, telles que la doxorubicine, ont un effet cardiotoxique qui résulterait de la production de FRO entraînant une peroxydation des structures lipidiques du réticulum sarcoplasmique et des mitochondries, et un dysfonctionnement de ces organites ; la bléomycine possède une forte toxicité pulmonaire, également attribuée à la production de FRO, et pouvant conduire à une fibrose pulmonaire interstitielle irréversible.

Différentes stratégies pour diminuer ces effets secondaires des traitements anti-cancéreux ont été proposées.

Dans le cas d'une cytotoxicité concernant plus particulièrement certains types cellulaires, il a été proposé d'utiliser des agents cytoprotecteurs, et notamment des agents capables de neutraliser les FRO, tels que la N-acétyl cystéine (DOROSHOW et al.. J. Clin. Invest., 68, 1053-1064, 1981) ou, plus récemment, la SOD ou des mimétiques de cette enzyme. Par exemple, la Demande PCT/WO 97/49390 propose d'utiliser un chélate de manganèse dérivé de dipyrydoxal, le MnDPDP, pour prévenir les effets cardiotoxiques des anthracyclines ; la Demande PCT/WO 02/060383 rapporte la capacité de deux chélates de manganèse dérivés de porphyrine, le MnTBAP et le MnTM-4-PyP, à protéger les cellules de l'épithélium pulmonaire des effets toxiques de la radiothérapie et de la bléomycine ; cette Demande rapporte également que ces dérivés sont capables d'inhiber sélectivement la prolifération de cellules d'adénocarcinome pulmonaire, sans affecter celle de cellules épithéliales ou endothéliales normales.

La demande WO 84/04922 A décrit l'activité anti-tumorale du mimétique de superoxyde dismutase CuDIPS, éventuellement en combinaison avec d'autres agents anti-tumoraux.

Pour réduire les conséquences des effets cytotoxiques des molécules anti-cancéreuses vis-à-vis des cellules hématopoïétiques, on utilise généralement des facteurs de croissance hématopoïétiques, afin de réduire la durée de la leucopénie et le risque infectieux qui en découle. L'utilisation d'agents cytoprotecteurs est limitée par le risque du manque de sélectivité de ces agents, du fait du taux de division rapide des cellules hématopoïétiques. A l'heure actuelle, le seul agent cytoprotecteur utilisé pour réduire la leucopénie est l'amifostine, qui est un précurseur phosphorylé d'un antioxydant à groupement thiol, et dont la sélectivité résulte de sa pénétration préférentielle dans les cellules non-tumorales où il libère la molécule active.

Les Inventeurs ont entrepris de tester les effets de différentes molécules, connues pour leur capacité de neutraliser à différents niveaux la production de FRO, sur la prolifération de différentes lignées de cellules tumorales, ainsi que sur la viabilité de ces cellules tumorales et celle de leucocytes humains normaux ; ils ont ensuite testé, de la même manière, les effets de ces molécules sur les propriétés cytostatiques et cytotoxiques d'agents de chimiothérapie antitumorale connus pour induire la production de FRO.

Les molécules antioxydantes qui ont été testées sont les suivantes :
- la N-acétyl cystéine (NAC) (référence), qui est un antioxydant, capteur de radicaux libres, et précurseur du glutathion intracellulaire ;
- le CuDIPS (Cu[II]-[diisopropylsalicylate]) (référence) qui est un mimétique chimique de la CuZn SOD (MC KENZIE et al., Br. J. Pharmacol. 127, 1159-1164, 1999) ;
- le MnTBAP (Mn(III) tetrakis (5,10,15,20-benzoic acid) porphyrine) (référence), qui est un mimétique chimique de la MnSOD (PASTERNACK et al., Inorg. Biochem., 15, 261-267 1981) ainsi que de la catalase et de la glutathion peroxydase (Demande PCT/WO 01/12327) ;
- le MnDPDP (manganèse dipyridoxyl phosphate (Mn-DPDP) également dénommé Mangafodipir (DCI), qui est un mimétique chimique de la MnSOD ainsi que de la catalase et de la glutathion réductase (Demande PCT/WO 02/087579).

Les Inventeurs ont observé que le traitement par la NAC induit une augmentation de la prolifération des cellules tumorales, alors que le traitement par le MnTBAP, le CuDIPS, ou le MnDPDP induit une réduction de cette prolifération. En ce qui concerne la viabilité cellulaire, la NAC n'a aucun effet sur celle-ci, qu'il s'agisse des cellules tumorales ou des leucocytes humains normaux. Le MnTBAP ou le CuDIPS diminuent la viabilité des cellules tumorales et également, bien que dans une moindre mesure, celle des leucocytes humains normaux. En revanche, le MnDPDP diminue la viabilité des cellules tumorales, mais, de manière surprenante, n'influence pas celle des leucocytes humains normaux.

Dans le cas de l'association de ces molécules antioxydantes avec des agents antitumoraux, les Inventeurs ont observé que la NAC inhibe les effets cytostatiques et cytotoxiques de ces agents sur les cellules tumorales, alors que le MnTBAP, le CuDIPS, et le MnDPDP les augmentent.

Les effets de la NAC, du MnTBAP, et du CuDIPS sur la cytotoxicité des agents antitumoraux vis-à-vis des leucocytes normaux sont similaires à ceux observés sur les cellules tumorales ; en revanche le MnDPDP diminue la cytotoxicité des agents antitumoraux sur les leucocytes humains normaux, à l'inverse de l'effet observé dans le cas des cellules tumorales.

Il apparaît donc que le MnDPDP est capable d'induire ou de potentialiser un stress oxydant chimio-induit au niveau des cellules tumorales, tout en préservant la viabilité des leucocytes normaux.

Les Inventeurs ont également testé les effets de la NAC, du MnTBAP, du CuDIPS et du MnDPDP, administrés isolément ou associés à un agent de chimiothérapie antitumorale, sur le développement de tumeurs *in vivo* chez la souris.

Ils ont observé que l'administration de NAC induisait une augmentation du volume tumoral, alors que l'administration de MnTBAP, de CuDIPS ou de MnDPDP diminue le volume des tumeurs. En association avec un agent antitumoral, la NAC bloque l'effet inhibiteur de cet agent sur la croissance tumorale, alors que le MnTBAP, le CuDIPS ou le MnDPDP augmentent cet effet inhibiteur.

Ces propriétés singulières du mangafodipir, par rapport à celles d'autres anti-oxydants, et en particuliers des autres mimétiques de SOD testés, apparaissent liées à sa double activité de mimétique de la superoxyde dismutase et de la glutathion réductase.

La présente invention a pour objet l'utilisation du Mangafodipir (MnDPDP) en tant que principe actif antitumoral et protecteur des leucocytes, pour l'obtention d'un médicament destiné à un traitement anti-cancéreux.

Selon un mode de mise en oeuvre préféré de la présente invention, le Mangafodipir est utilisé en association avec un autre agent anti-tumoral, choisi parmi la doxorubicine, la mitomycine C, l'étoposide, les dérivés du platine, le tamoxifène, les taxanes, le 5-fluoro-uracile, l'irinotécan (inhibiteur de la topo-isomérase-1), la gemcitabine (anti-métabolite), l'endoxan (agent électrophile alkylant), la streptozotocine (agent électrophile non-alkylant), la bléomycine (agent scindant l'ADN), et la vincristine (poison du fuseau).

Du fait de la simultanéité de leur effet cytotoxique et cytostatique vis-à-vis des cellules tumorales, et de leur effet protecteur vis-à-vis des leucocytes normaux, le Mangafodipir permet d'accroître significativement l'index thérapeutique des médicaments anticancéreux auxquels il est associé. En effet, il exerce avec ces médicaments anti-cancéreux une action synergique antitumorale, tout en protégeant les leucocytes des effets délétères de la chimiothérapie.

La présente invention a également pour objet une composition pharmaceutique comprenant du Mangafodipir associé avec un autre agent anti-tumoral, tel que défini dans la revendication 3.

Pour la mise en oeuvre de la présente invention, le Mangafodipir sera généralement employé dans des formulations permettant l'administration d'une dose de principe actif comprise entre 1 et 100 mg/kg/jour. Des doses plus élevées peuvent toutefois être utilisées, compte tenu de la faible toxicité de ce produit. Il est bien entendu que l'homme de l'art peut adapter ces doses en fonction des particularités de chaque patient et de la pathologie concernée.

Ces formulations peuvent être administrées par différentes voies, par exemple par voie orale, ou par injections, en particulier par injections sous-cutanées, intra-musculaires ou intra-veineuses. D'autres voies d'administration pourront être envisagées si elles augmentent l'efficacité, la biodisponibilité ou la tolérance des produits. La voie la plus appropriée peut être choisie par l'homme du métier en fonction de la formulation utilisée.

La présente invention sera mieux comprise à l'aide du complément de description qui va suivre, qui se réfère à des exemples montrant les propriétés antitumorales du Mangafodipir et ses effets cytoprotecteurs sur les leucocytes normaux.

### EXEMPLE 1 : INFLUENCE DE DIVERSES MOLECULES ANTIOXYDANTES SUR LES PROPRIETES PROLIFERATIVES BASALES DES CELLULES TUMORALES.

Des tests de prolifération cellulaire *in vitro*, ont été réalisés sur les lignées cellulaires suivantes : CT26 (mouse colon carcinoma, ATCC (American Type Culture Collection) n°2638), Hepa 1-6 (mouse liver hepatoma, ATCC n°1830), A 549 (Human lung carcinoma, ATCC n°185) Ces lignées ont été préalablement cultivées, dans un incubateur humide à 37°C sous 5% de CO2, en milieu Dulbecco's modified Eagle's medium (DMEM)/Glutamax-I contenant 10% de sérum de veau foetal et des antibiotiques [penicilline (100U/ml)/streptomycine (100µg/ml)] (LIFE TECHNOLOGIES, Cergy Pontoise, France). Toutes ces lignées cellulaires ont été testées régulièrement pour exclure toute infection à mycoplasmes.

Pour le test de prolifération, les cellules (2 x 10⁴ cellules/puits) ont été ensemencées dans des plaques 96 puits (COSTAR, Corning, Inc. NY, USA) et incubées 48 heures en milieu complet additionné de concentrations croissantes, de 0 à 400µm, de N-Acétyl-Cystéine (NAC, SIGMA, Saint-Quentin Fallavier, France), de MnTBAP (mimétique de la MnSOD ; CALBIOCHEM, Paris, France), de CuDIPS (mimétique de la Cu/Zn SOD ; SIGMA, Saint-Quentin Fallavier, France) ou de Mangafodipir (MnDPDP ou TELASCAN, AMERSHAM HEALTH, Amersham, UK). Le NAC, le MnTBAP et le CuDIPS n'entrent pas dans la protection revendiquée.

La prolifération cellulaire est déterminée en incubant les cellules pendant 16 heures avec de la [³H]-thymidine (1µCi/puits).

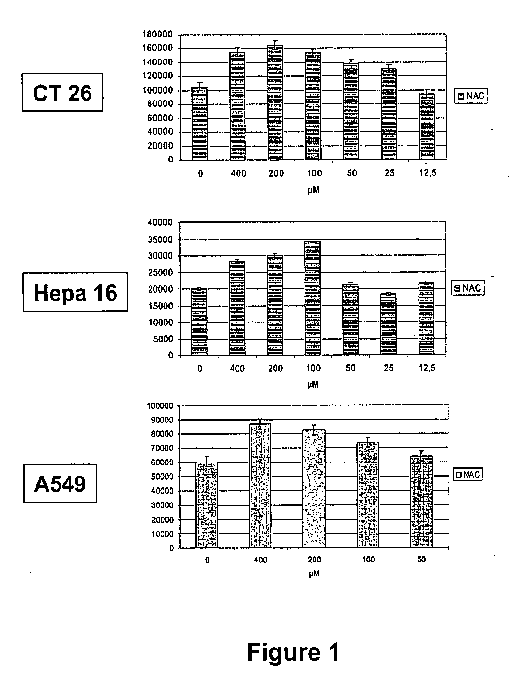

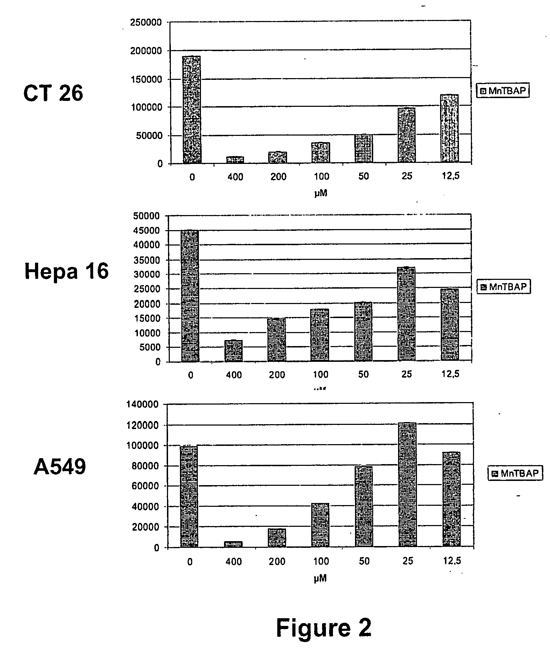

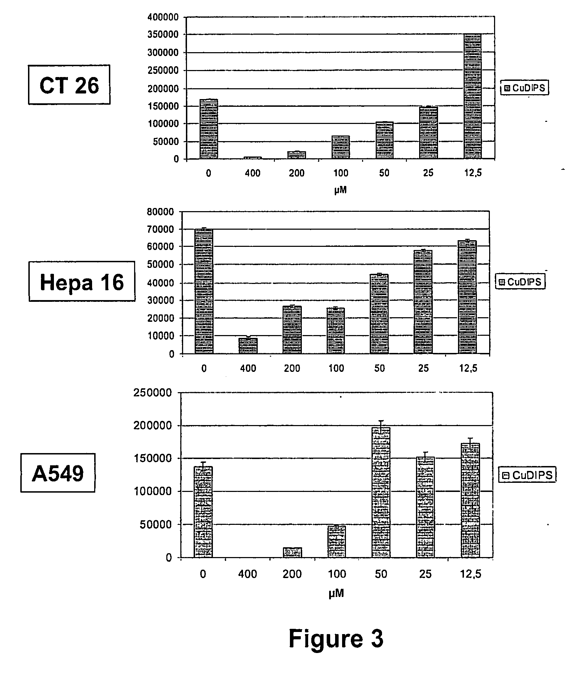

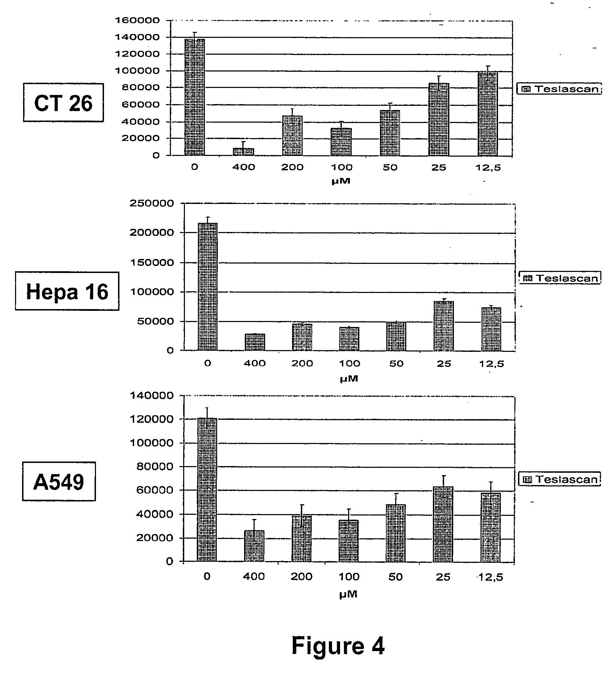

Les résultats de ces expériences, sur différentes lignées tumorales, pour la NAC, le MnTBAP, le CuDIPS et le MnDPDP sont illustrés dans les figures 1, 2, 3 et 4 respectivement.

Légende des figures 1, 2, 3, et 4 :
En abscisse : concentration en anti-oxydant (en µM),
En ordonnée : radioactivité de la [³H] thymidine en cpm.

On observe une augmentation de la prolifération des cellules tumorales en réponse au traitement par la NAC (figure 1). Cette augmentation de la prolifération est de 73% pour les cellules Hepa 1-6, en présence de 100µM de NAC et de 45 et 47% en présence de 400µM de NAC pour les cellules tumorales A 549 et CT26 respectivement.

Au contraire, le traitement des cellules tumorales Hepa 1-6, CT26 et A 549 avec le MnTBAP (figure 2), le CuDIPS (figure 3) ou le MnDPDP (TESLASCAN, figure 4) réduit de manière dose dépendante leur prolifération. Cette réduction de la prolifération cellulaire atteint près de 90% en présence de 400µM d'une de ces trois molécules.

### EXEMPLE 2 : EFFETS DE LA NAC, DU CuDIPS, DU MnTBAP ET DU MnDPDP SUR LA VIABILITE DE LIGNEES TUMORALES OU DE LEUCOCYTES HUMAINS NORMAUX.

Des tests de viabilité *in vitro*, en réponse au traitement avec la NAC, le CuDIPS, le MnTBAP ou le MnDPDP, ont été réalisés sur les lignées cellulaires de l'exemple 1 ainsi que sur des leucocytes humains normaux. Ces derniers ont été obtenus chez des volontaires sains, après consentement éclairé, par prélèvement de sang veineux recueilli sur anticoagulant (héparinate de lithium). Les globules rouges ont été lysés par choc osmotique à l'aide d'une solution hypotonique d'acétate de potassium et les leucocytes ont été cultivés dans les conditions décrites à l'exemple 1.
Pour les tests de viabilité, les cellules (2 × 10⁴ cellules/puits) ont été ensemencées dans des plaques 96 puits (COSTAR, Corning, Inc. NY, USA) et incubées 48 heures en milieu complet additionné de concentrations croissantes, de 0 à 400µm, de NAC, de MnTBAP, de CuDIPS ou de MnDPDP. La viabilité cellulaire a été évaluée par réduction d'un sel de méthylthiazol-tétrazolium (MTT ; SIGMA) en formazan. Les cellules ont été exposées à 20 µl de MTT (5 mg/ml en PBS) et incubées 4 h à 37°C. Puis, 150µl de milieu ont été retirés de chaque puits et la réaction est révélée par l'addition de 100µl de DMSO (SIGMA). L'absorbance est analysée pour chaque puits à 550nm et à 630nm avec un lecteur de plaque ELISA. Le nombre de cellules viables est déterminé, par la différence entre l'absorbance à 550 nm et l'absorbance à 630 nm.

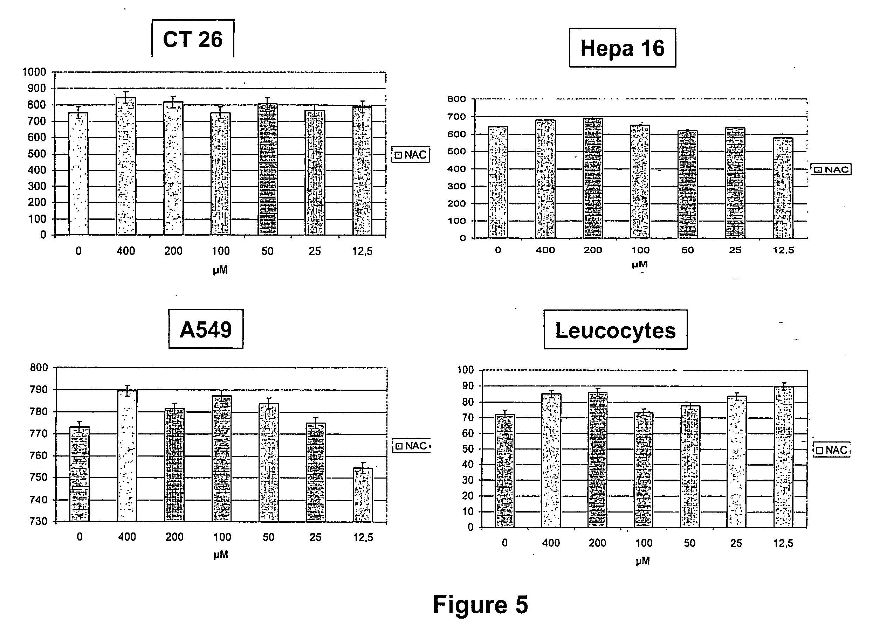

Les résultats de ces expériences pour les lignées tumorales CT26, Hepa 16 et A549, ainsi que pour les leucocytes normaux sont illustrés dans les figures 5, 6, 7 et 8, pour la NAC, le MnTBAP, le CuDIPS et le MnDPDP respectivement.

Légende des figures 5 à 8 :
En abscisse : concentration en anti-oxydant (en µM),
En ordonnée : DO à 550 nm - DO à 630 nm.

On observe que le traitement par la NAC des cellules tumorales Hepa 1-6, CT26 et A 549 ou des leucocytes humains normaux est sans effet sur la viabilité cellulaire (figure 5).

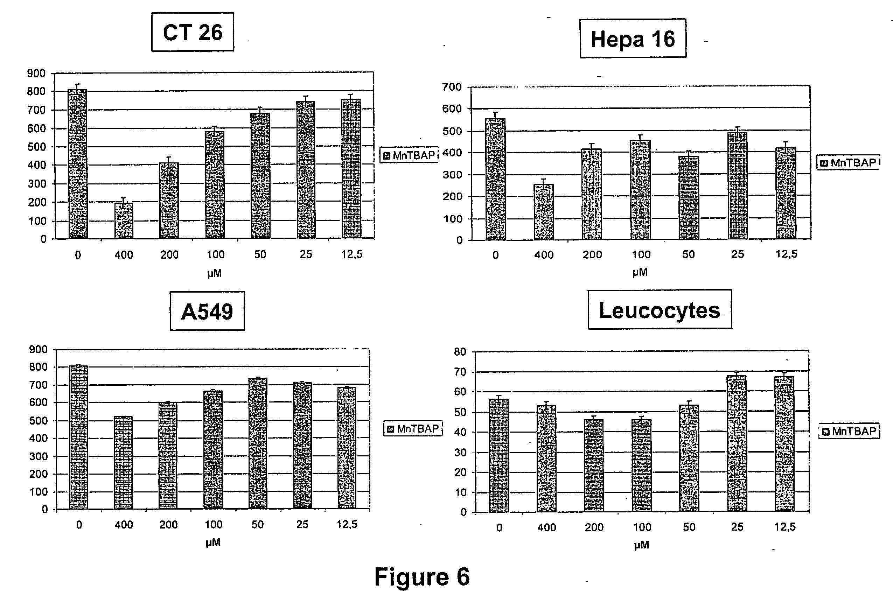

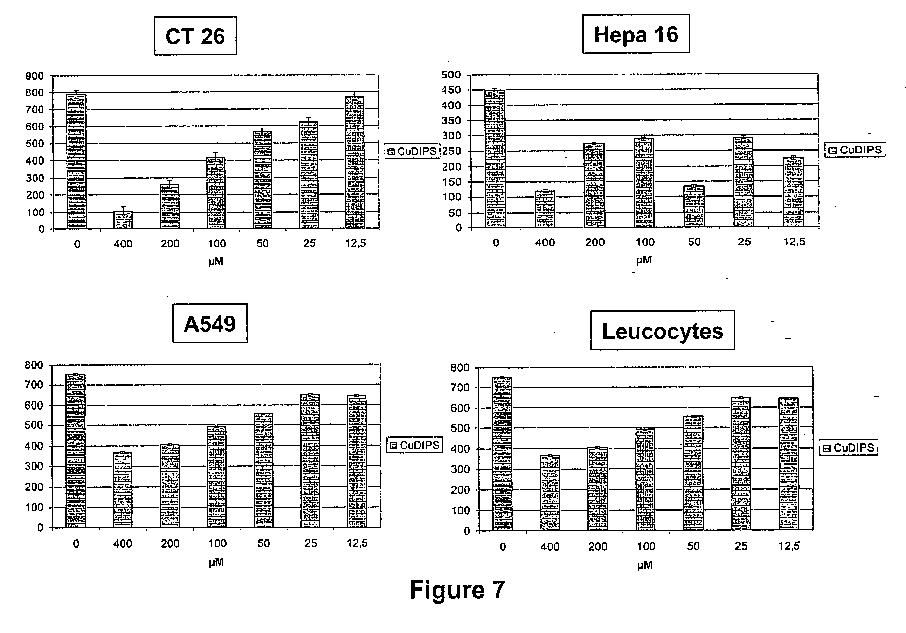

Au contraire, le traitement des cellules tumorales Hepa 1-6, CT26 et A 549 avec le MnTBAP (figure 6) ou le CuDIPS (figure 7) diminue de manière dose dépendante la viabilité des cellules tumorales. La viabilité des cellules tumorales Hepa 1-6, CT26 et A 549 est réduite de 62%, 75% et 37%, respectivement, par 400µM MnTBAP, et de 74%, 85% et 50% respectivement, par 400µM de CuDIPS. Toutefois, le traitement des leucocytes humains normaux avec le MnTBAP et le CuDIPS induit également une diminution de la viabilité cellulaire, laquelle atteint au maximum 18% et 50% respectivement.

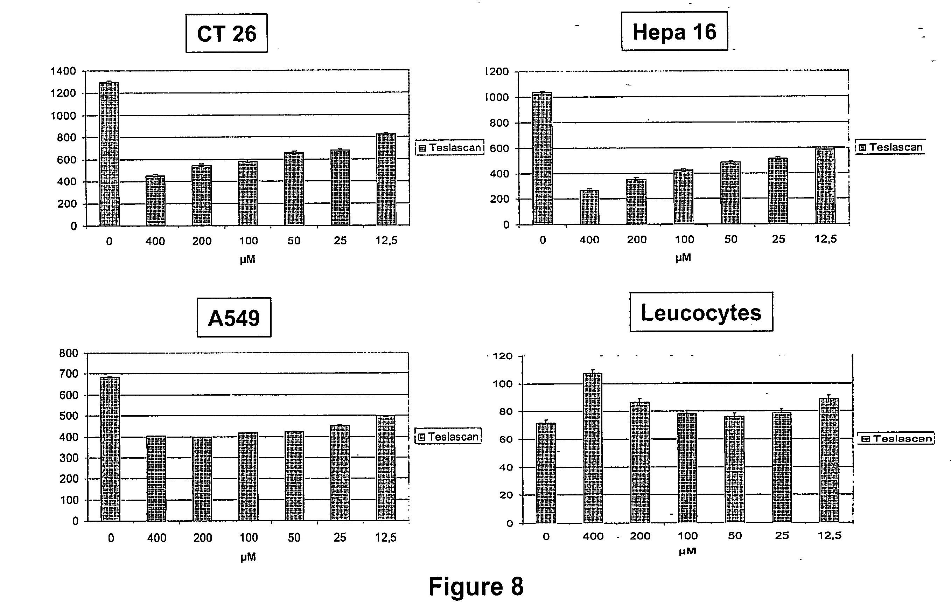

Enfin, si le MnDPDP (Mangafodipir ou TESLASCAN, figure 8) réduit également de manière dose dépendante la viabilité des cellules tumorales Hepa 1-6, CT26 et A 549, il n'influence pas la viabilité des leucocytes humains normaux, et ceci quelle que soit la dose de mangafodipir utilisée.

### EXEMPLE 3 : EFFETS DE LA NAC, DU CuDIPS, DU MnTBAP ET DU MnDPDP SUR LES PROPRIETES ANTI-PROLIFERATIVES ET CYTOTOXIQUES DE MOLECULES UTILISEES DANS LA CHIMIOTHERAPIE DES CANCERS.

Les molécules antitumorales suivantes : oxaliplatine (appartenant à la famille du cisplatine) ; taxol ; 5-fluoro-uracile ; qui sont connues pour induire la production de FRO dans les cellules tumorales, ont été utilisées. Pour chacune de ces molécules, des tests de prolifération et de viabilité cellulaire ont été effectués, en l'absence de molécules antioxydantes, ou en présence de concentrations croissantes de NAC, de MnTBAP, de CuDIPS ou de MnDPDP.

### 1) Effets sur les propriétés antiprolifératives

Les tests de prolifération ont été effectués sur les lignées tumorales CT26, Hepa 16, et A549, selon le protocole décrit à l'exemple 1.

### Oxaliplatine :

L'oxaliplatine (ELOXATINE ou [(1R,2R)-1,2-cyclohexanediamine-N,N'] [oxalato (2-)-O,O']platine (II) ; SANOFI-PHARMA, Paris, France) a été utilisé dans tous les tests à une concentration de 10 µM.

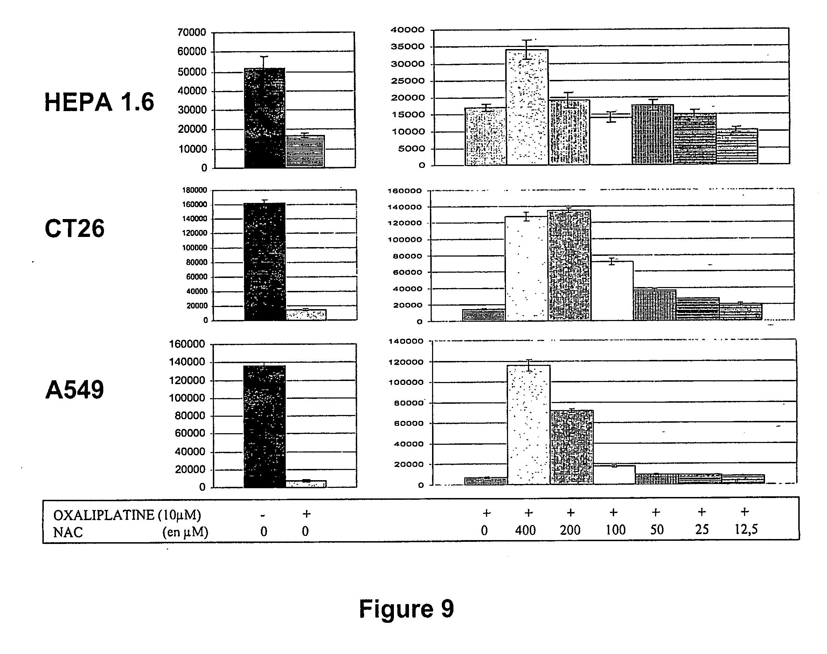

Les résultats des tests de prolifération cellulaire des lignées tumorales CT26, Hepa 16 et A549 sont illustrés dans les figures 9, 10, 11 et 12, pour la NAC, le MnTBAP, le CuDIPS et le MnDPDP respectivement.

Légende des figures 9 à 12 :
En abscisse : présence (+) ou absence (-) d'oxaliplatine ; concentration en anti-oxydant (en µM),
En ordonnée : radioactivité de la [³H] thymidine en cpm.

Le traitement des lignées tumorales Hepa 1-6, CT26 et A549 avec 10µM d'oxaliplatine seul diminue la prolifération des cellules tumorales de 70%, 91% et 93% respectivement (figure 9 à 12).

La NAC réduit de manière dose dépendante l'effet cytostatique de l'oxaliplatine et ceci quel que soit le type de cellules tumorales (figure 9).

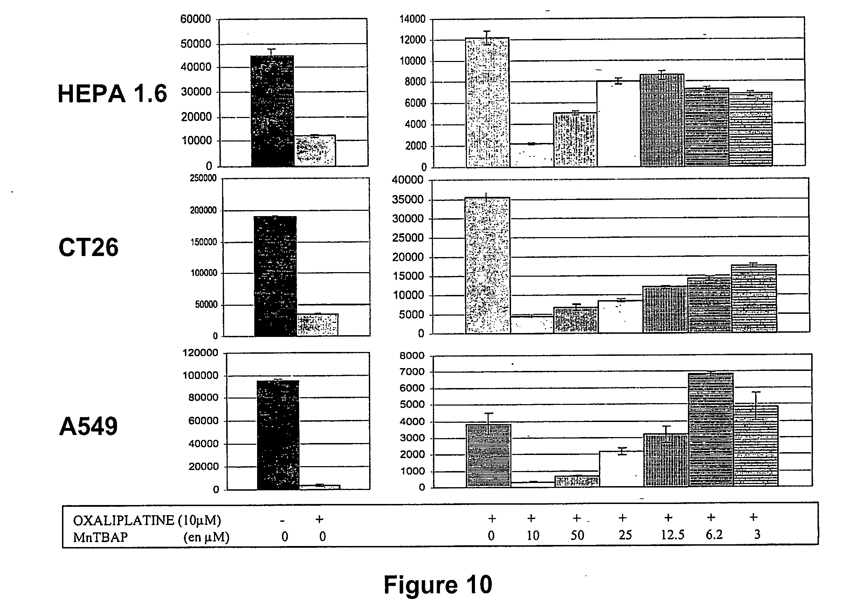

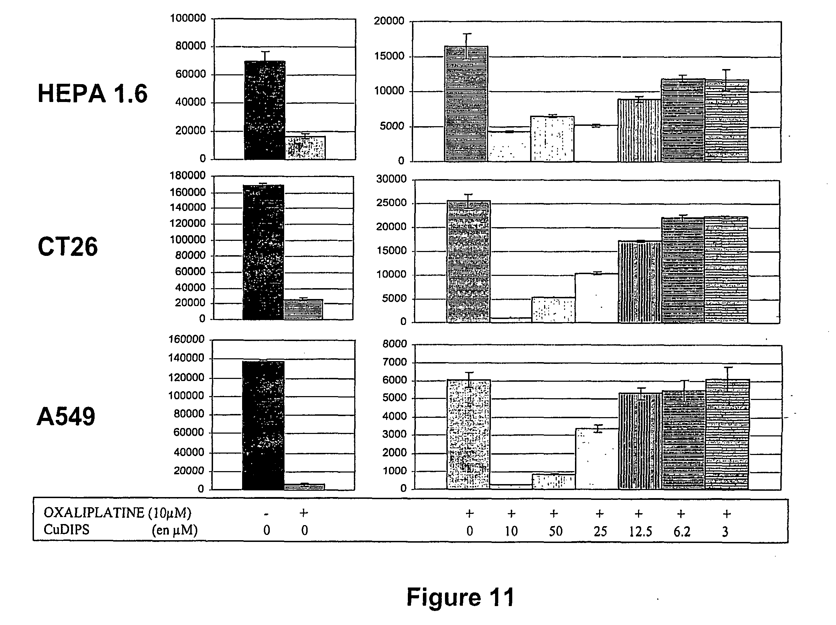

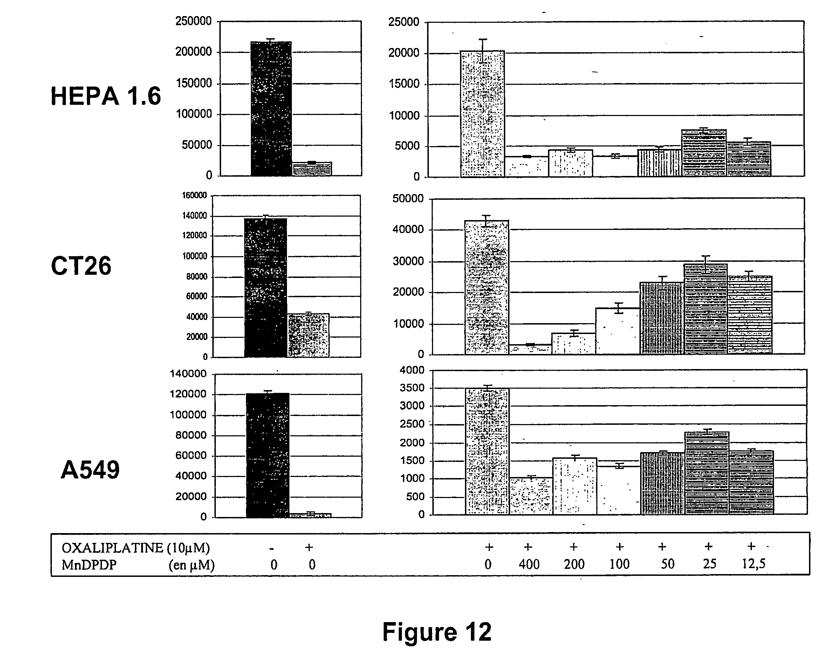

Au contraire, le MnTBAP (figure 10), le CuDIPS (figure 11) et le MnDPDP (figure 12) augmentent de manière dose-dépendante les propriétés anti-prolifératives de l'oxaliplatine.

### Taxol :

Le taxol (PACLITAXEL ; BRISTOL-MYERS-SQUIBB, Paris, France) a été utilisé dans tous les tests à une concentration de 10 µM.

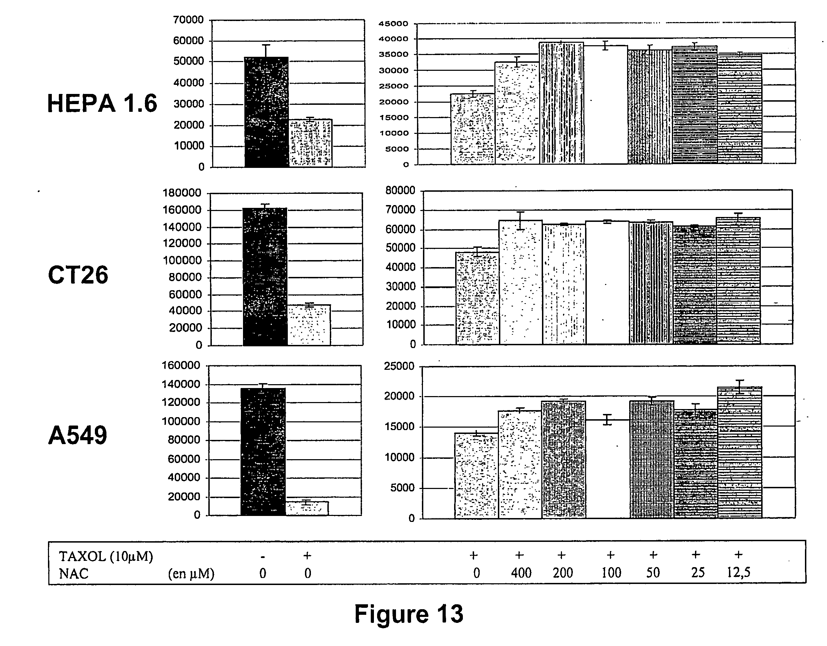

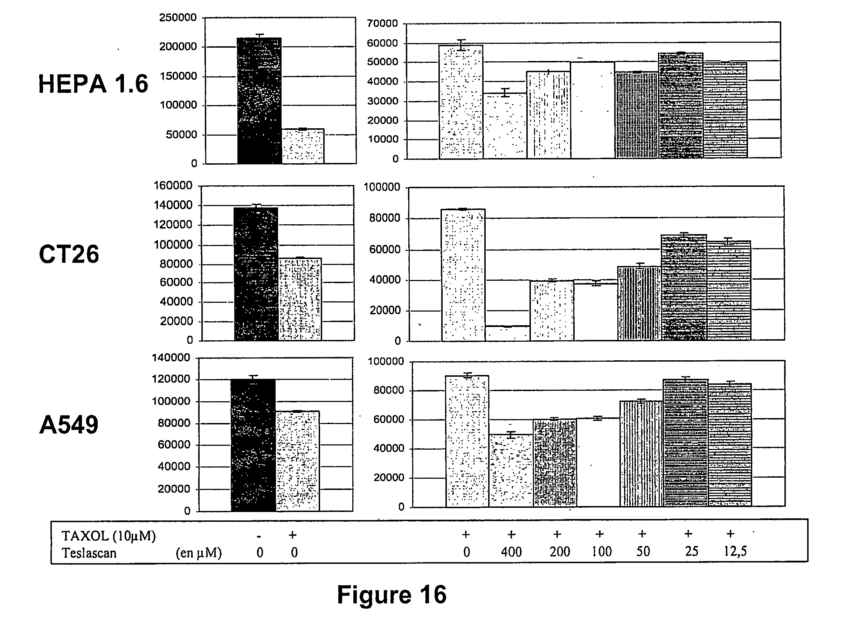

Les résultats des tests de prolifération des lignées tumorales CT26, Hepa 16 et A549 sont illustrés dans les figures 13, 14, 15 et 16, pour la NAC, le MnTBAP, le CuDIPS et le MnDPDP respectivement.

Légende des figures 13 à 16 :
En abscisse : présence (+) ou absence (-) de taxol ; concentration en anti-oxydant (en µM)
En ordonnée : radioactivité de la [³H] thymidine en cpm.

L'incubation avec le taxol réduit respectivement la prolifération des cellules tumorales A549, CT26 ou Hepa 1-6 de 85%, 71% et 65%, (figure 13 à 16) .

L'addition de NAC réduit de manière dose-dépendante l'effet cytostatique du taxol sur les cellules tumorales (figure 13).

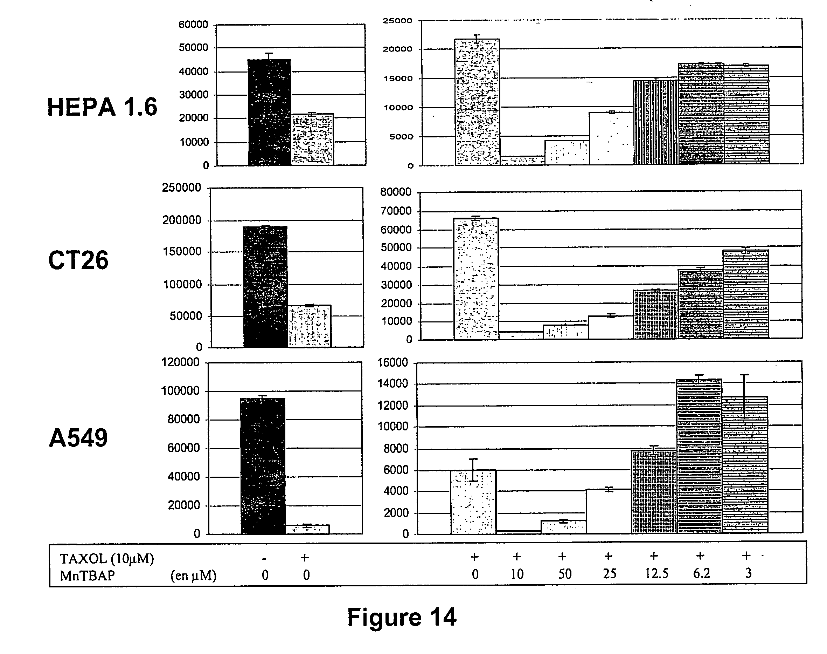

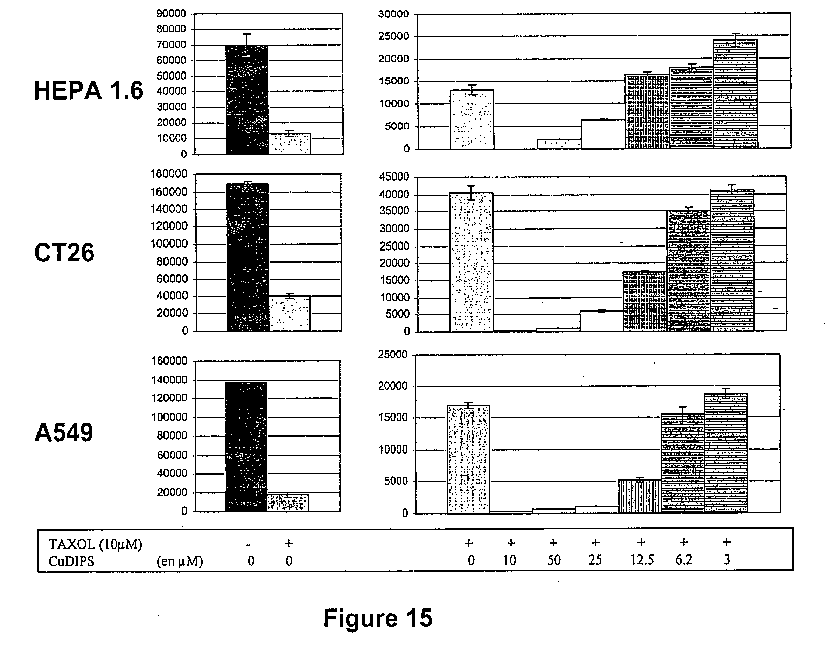

Au contraire, l'addition des trois mimétiques de SOD [MnTBAP (figure 14), CuDIPS (figure 15) ou de MnDPDP (figue 16)] augmente l'effet cytostatique du taxol de manière dose-dépendante.

### 5-FluoroUracil (5-FU):

Le 5-FluoroUracil (5-FU) (fluoro-5 tétrahydro-1,2,3,4 pyrimidinedione-2,5 ou fluoro-uracile ; ICN PHARMACEUTICAL FRANCE, Orsay, France) a été utilisé dans tous les tests à une concentration de 50 µM.

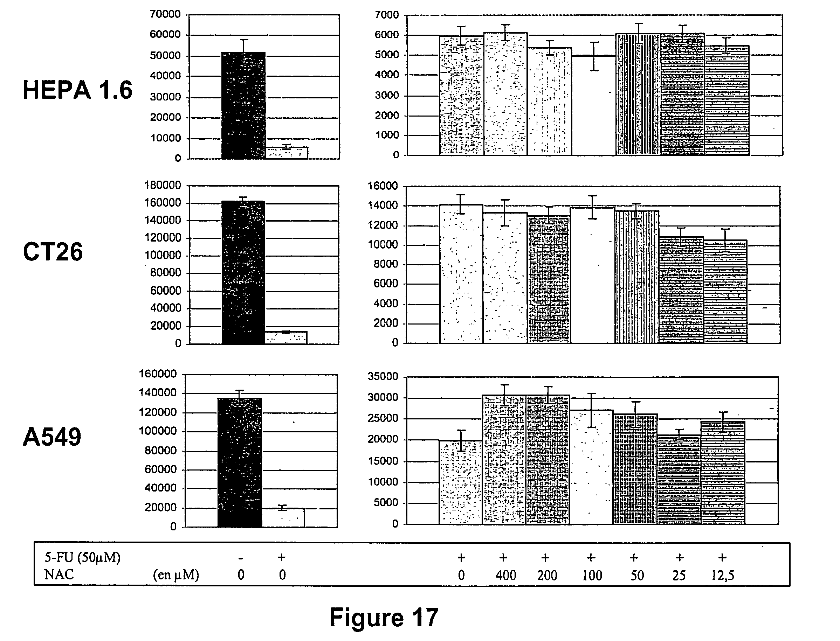

Les résultats des tests de prolifération des lignées tumorales CT26, Hepa 16 et A549 sont illustrés dans les figures 17, 18, 19 et 20, pour la NAC, le MnTBAP, le CuDIPS et le MnDPDP respectivement.

Légende des figures 17 à 20 :
En abscisse : présence (+) ou absence (-) de 5-FU ; concentration en anti-oxydant (en µM),
En ordonnée : radioactivité de la [³H] thymidine en cpm.

L'incubation des cellules tumorales avec le 5-FU réduit la prolifération des cellules tumorales Hepa 1-6, CT26 et A549 de 91%, 91% et 85%, respectivement (figure 17 à 20).

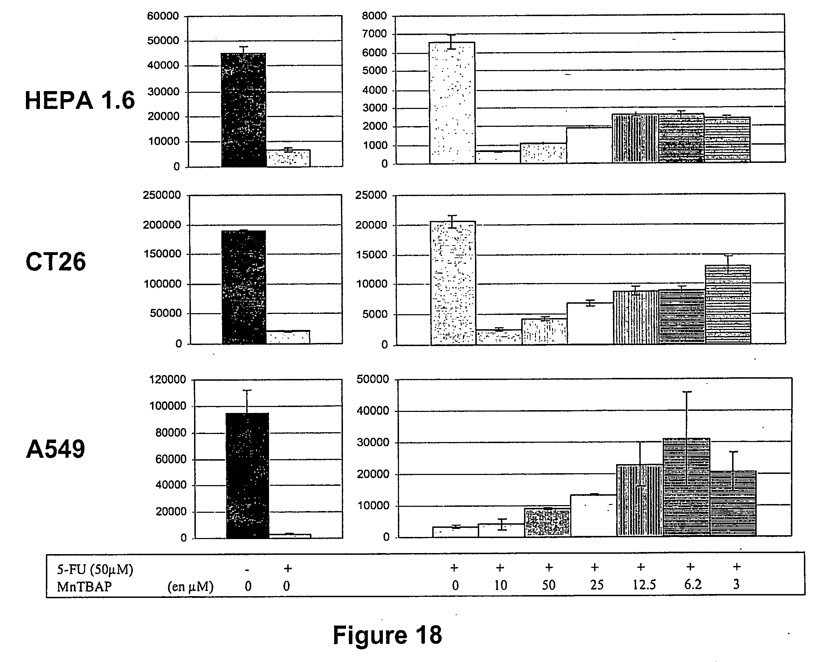

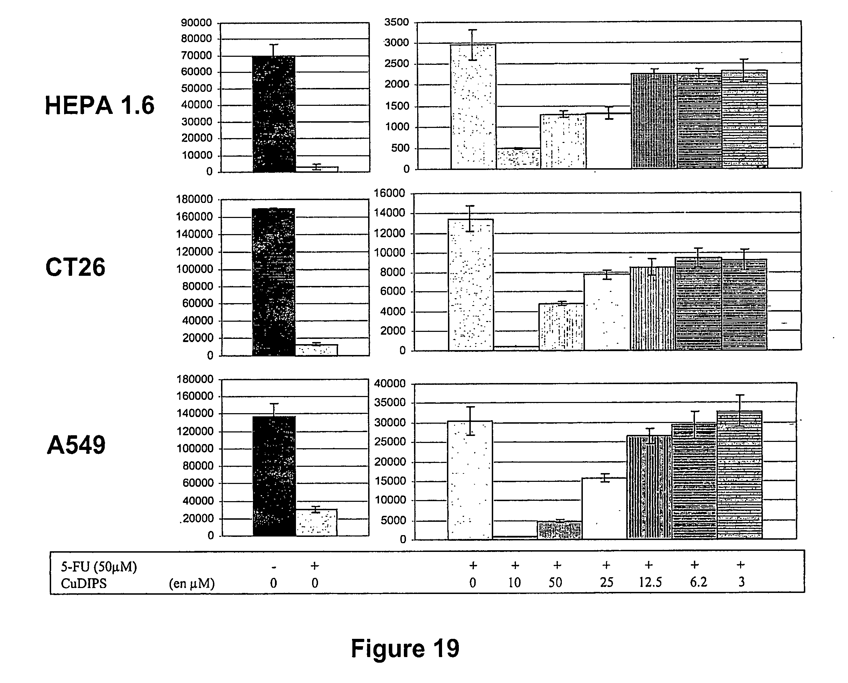

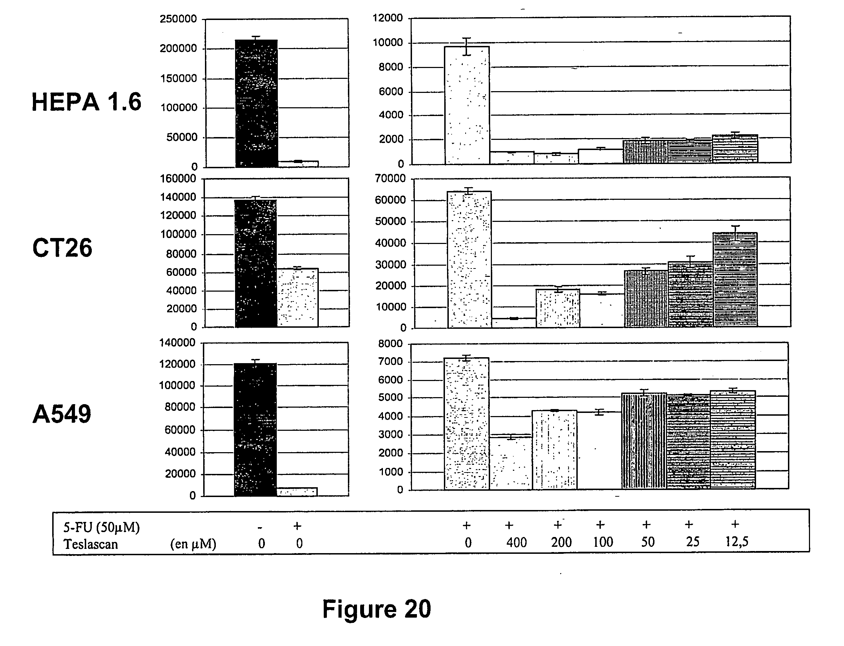

Comme pour l'oxaliplatine et le TAXOL, La NAC inhibe l'effet cytostatique du 5-FU sur les cellules tumorales (figure 17) alors que les trois mimétiques de SOD [MnTBAP (figure 18), CuDIPS (figure 19) et MnDPDP (TESLASCAN, figure 20)] l'augmentent.

### 2) Effets sur la viabilité cellulaire :

Les tests de viabilité ont été effectués sur les lignées tumorales CT26, Hepa 16, et A549, ainsi que sur des leucocytes humains normaux, selon le protocole décrit à l'exemple 2.

### Oxaliplatine :

L'oxaliplatine a été utilisé à une concentration de 10 µM dans le cas des cellules tumorales, et à une concentration de 1 mM dans le cas des leucocytes normaux.

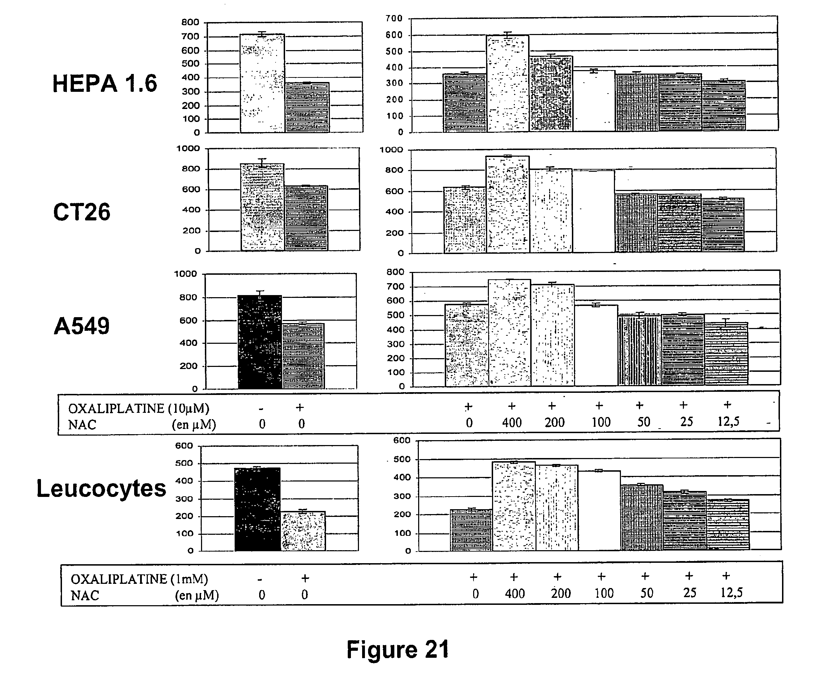

Les résultats sont illustrés par les figures 21, 22, 23 et 24, pour la NAC, le MnTBAP, le CuDIPS et le MnDPDP respectivement.

Légende des figures 21 à 24 :
En abscisse : présence (+) ou absence (-) d'oxaliplatine ; concentration en antioxydant (en µM),
En ordonnée : DO à 550 nm - DO à 630 nm.

Le traitement par l'oxaliplatine seul diminue en moyenne la viabilité des cellules tumorales Hepa 1-6, CT26 et A549 de 50%, 27% et 28% respectivement, et celle des leucocytes normaux d'environ d'environ 50% (figures 21 à 24).

La NAC diminue de manière dose-dépendante les effets cytotoxiques de l'oxaliplatine sur tous les types de cellules tumorales, ainsi que sur les leucocytes normaux (figure 21).

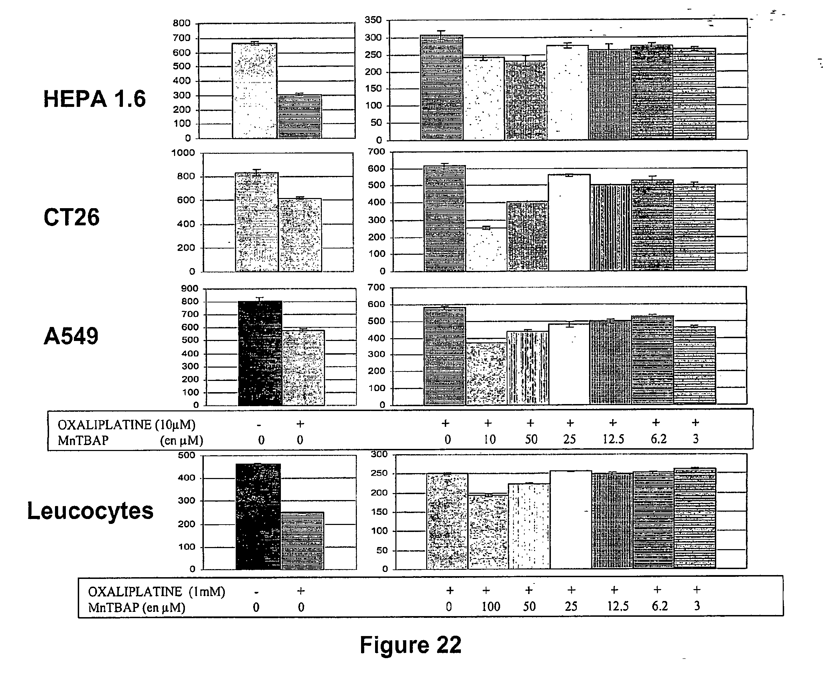

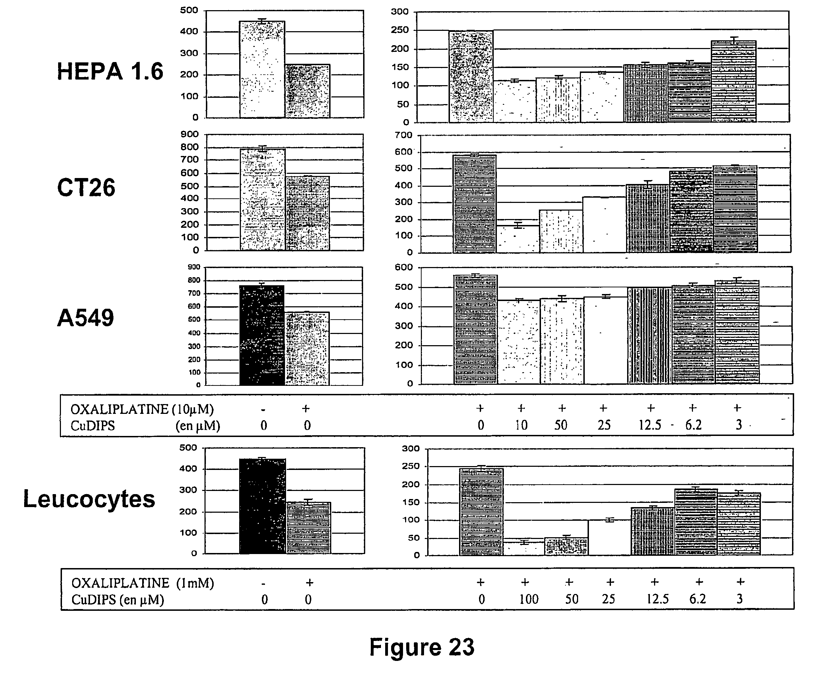

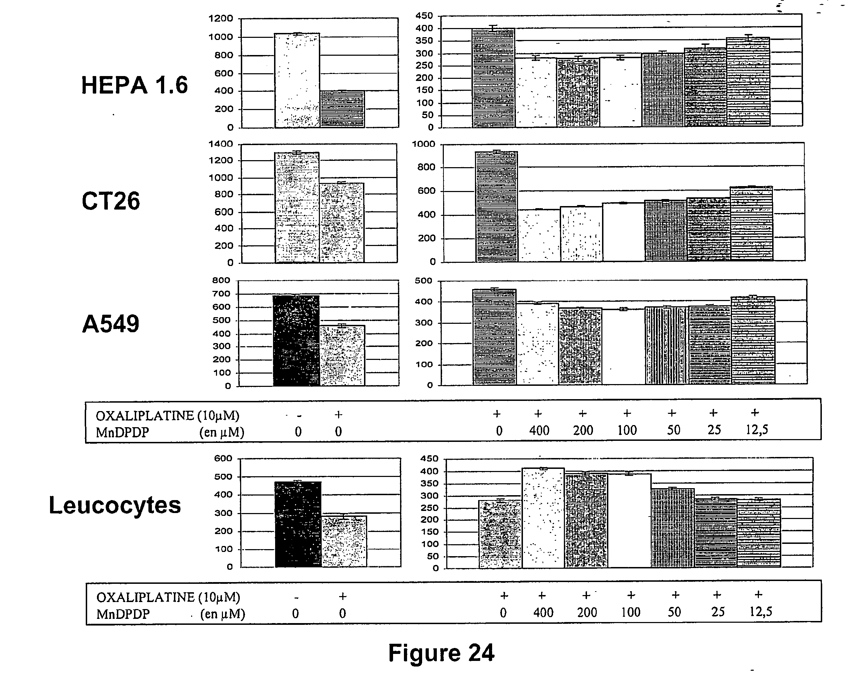

Le MnTBAP (figure 22), le CuDIPS (figure 23) et le MnDPDP (figure 24) augmentent de manière dose-dépendante les propriétés cytotoxiques de l'oxaliplatine sur les cellules tumorales.

Sur les leucocytes normaux, le MnTBAP(figure 22) et le CuDIPS (figure 23), augmentent également les propriétés cytotoxiques de l'oxaliplatine ; en revanche, le MnDPDP (figure 24) inhibe comme la NAC l'effet cytotoxique de l'oxaliplatine.

### Taxol :

Le taxol a été utilisé à une concentration de 10 µM dans le cas des cellules tumorales, et à une concentration de 20 µM dans le cas des leucocytes normaux.

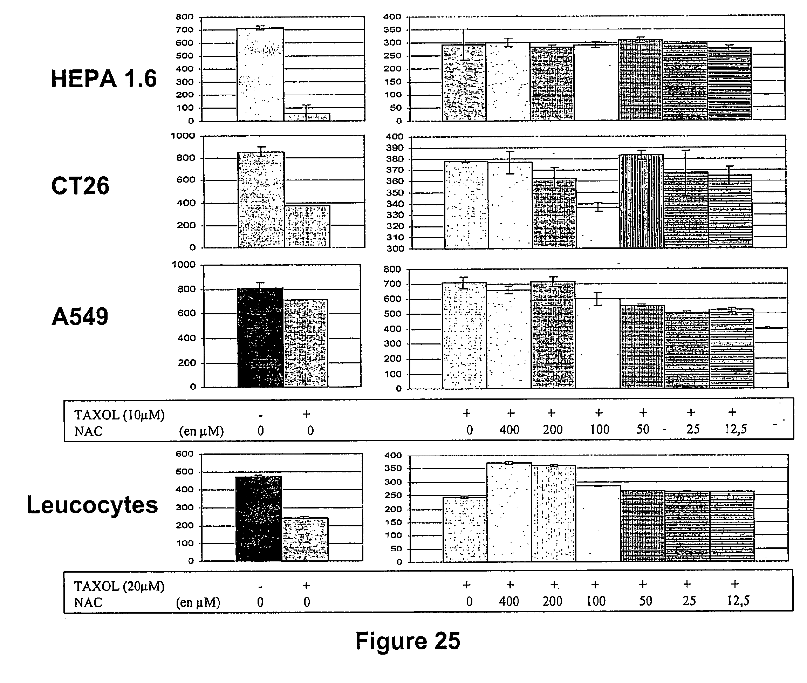

Les résultats sont illustrés par les Figures 25, 26, 27 et 28, pour la NAC, le MnTBAP, le CuDIPS et le MnDPDP respectivement.

Légende des figures 25 à 28 :
En abscisse : présence (+) ou absence (-) de taxol ; concentration en anti-oxydant (en µM),
En ordonnée : DO à 550 nm - DO à 630 nm.

Le traitement par le taxol seul diminue, en moyenne, la viabilité des cellules tumorales Hepa 1-6, CT26 et A549 de respectivement 25%, 50% et 47% en moyenne, et celle des leucocytes normaux d'environ 50% (Figures 25 à 28).

L'addition de NAC n'influence pas l'activité cytotoxique du taxol sur les cellules tumorales, et la diminue sur les leucocytes normaux (Figure 25).

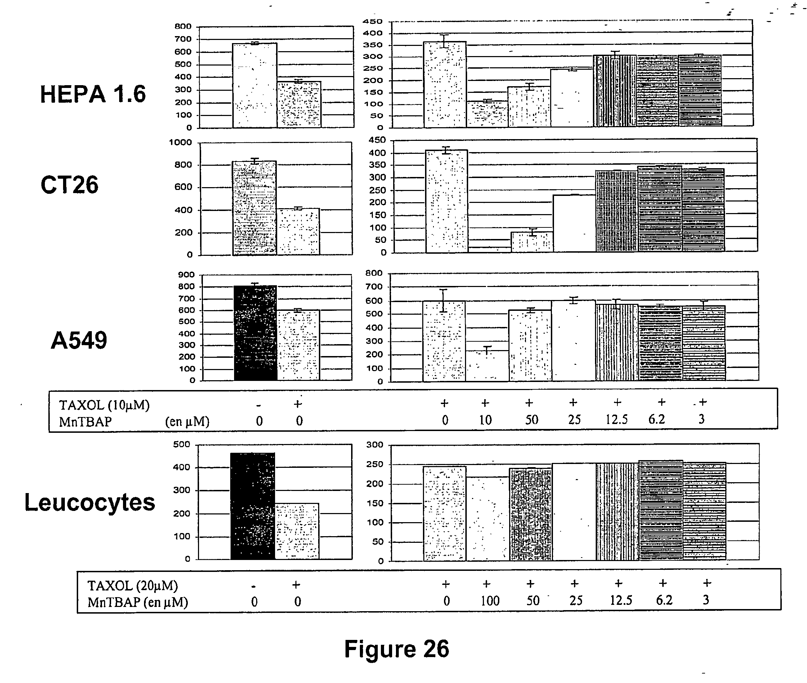

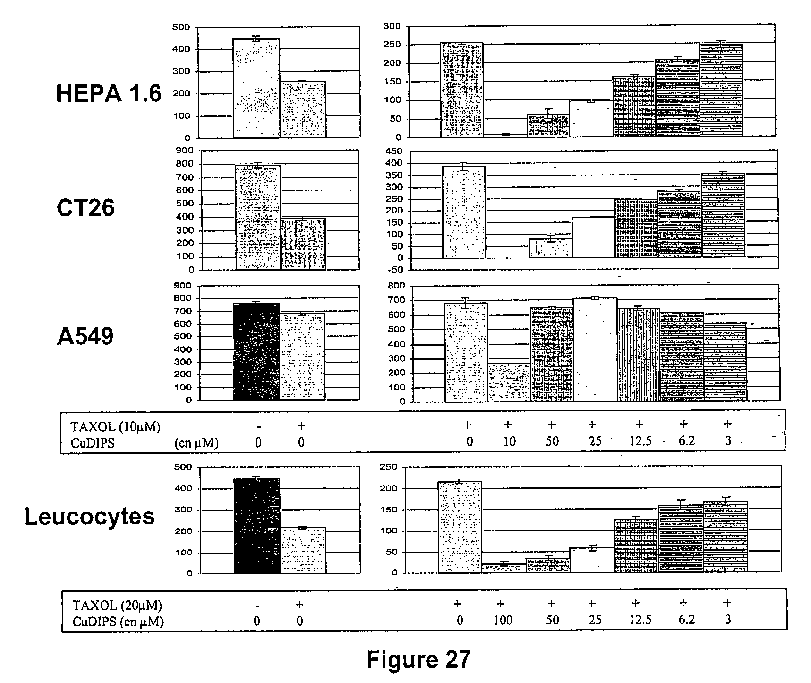

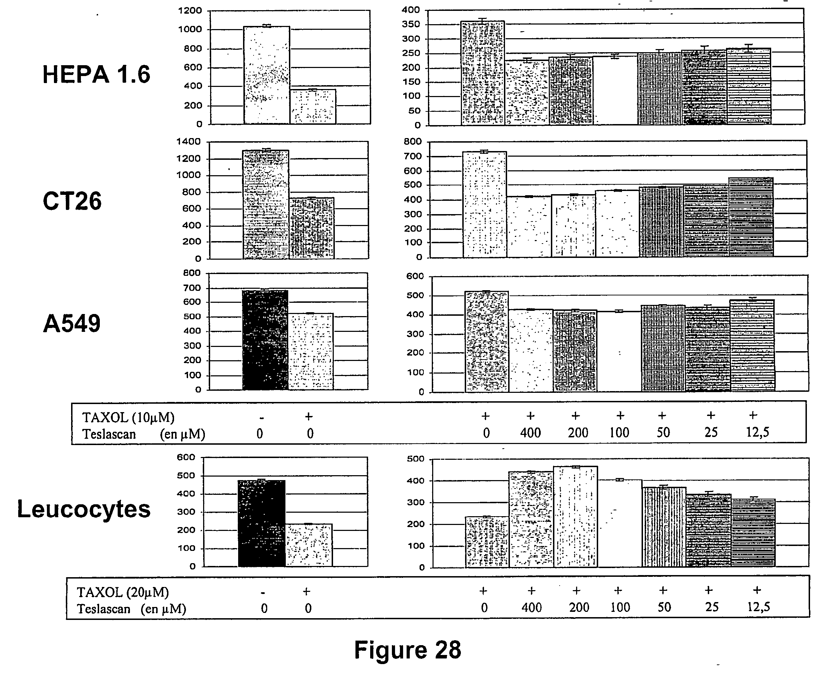

L'addition de MnTBAP (Figure 26), de CuDIPS (Figure 27) ou de MnDPDP (Figure 28)) augmente l'activité cytotoxique du taxol sur les cellules tumorales. Sur les leucocytes normaux, le MnTBAP n'a pratiquement pas d'influence sur l'effet cytotoxique du taxol (figure 26) et le CuDIPS (figure 27), augmente cet effet cytotoxique ; en revanche, le MnDPDP (figure 28) inhibe comme la NAC l'effet cytotoxique du taxol.

### 5-FluoroUracil (5-FU):

Le 5-FU a été utilisé à une concentration de 50 µM dans le cas des cellules tumorales, et à une concentration de 40 mM dans le cas des leucocytes normaux.

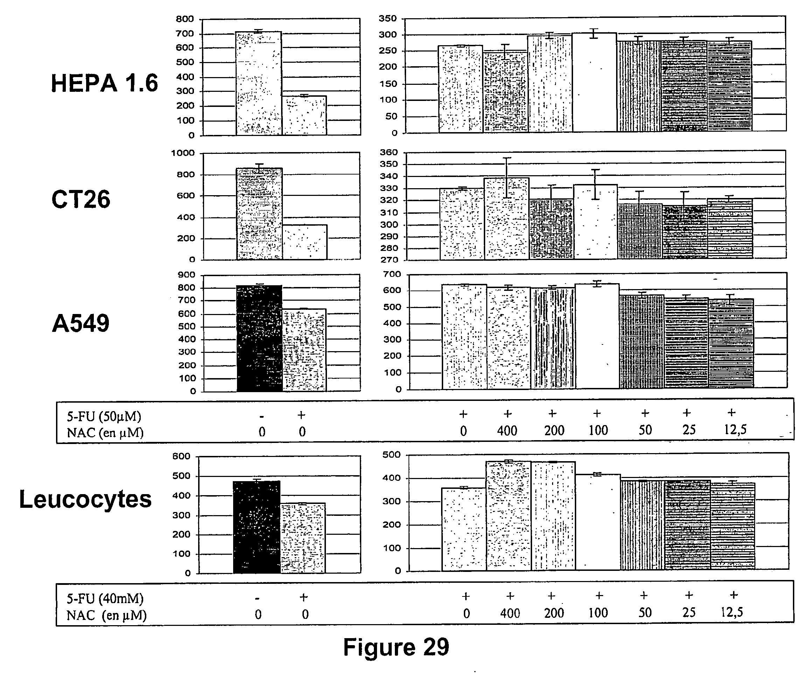

Les résultats sont illustrés par les Figures 29, 30, 31 et 32, pour la NAC, le MnTBAP, le CuDIPS et le MnDPDP respectivement.

Légende des figures 29 à 32 :
En abscisse : présence (+) ou absence (-) de 5-FU ; concentration en anti-oxydant (en µM),
En ordonnée : DO à 550 nm - DO à 630 nm.

Le traitement par le 5-FU seul diminue, en moyenne, la viabilité des cellules tumorales Hepa 1-6, CT26 et A549 de respectivement 65%, 85% et 25%, et celle des leucocytes normaux d'environ 19% (figures 29 à 32).

L'addition de NAC ne modifie pas l'activité cytotoxique du 5-FU sur les cellules tumorales, et la diminue sur les leucocytes normaux (Figure 29).

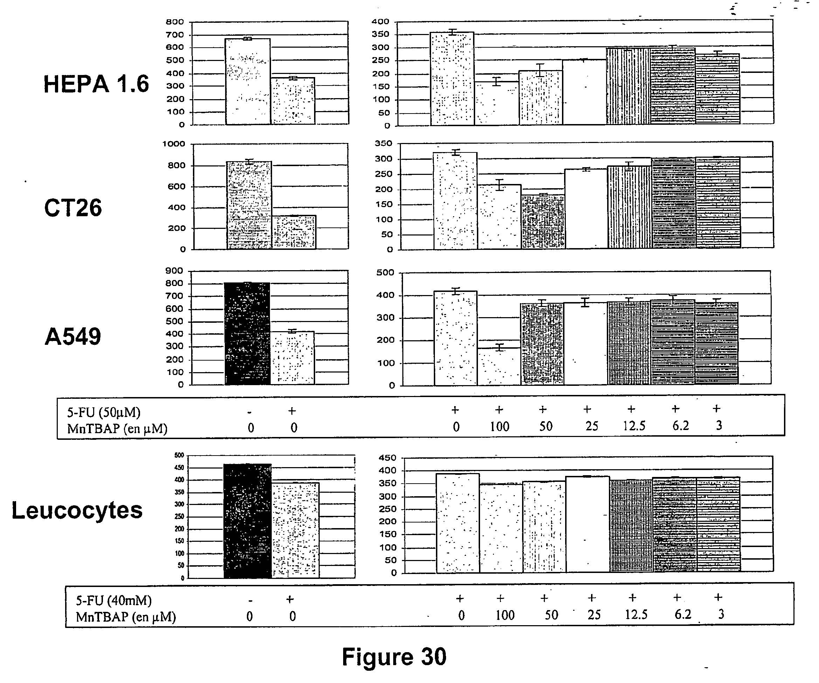

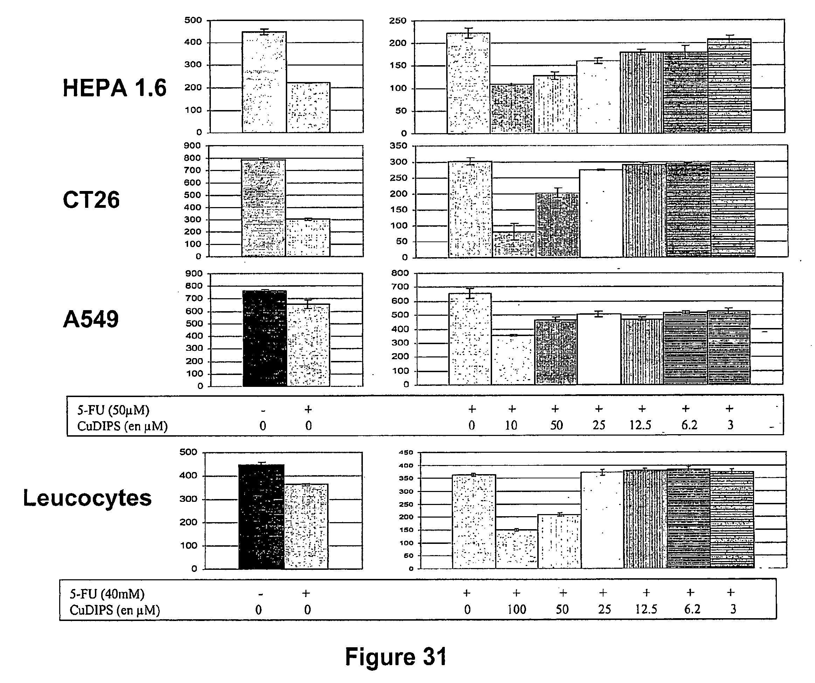

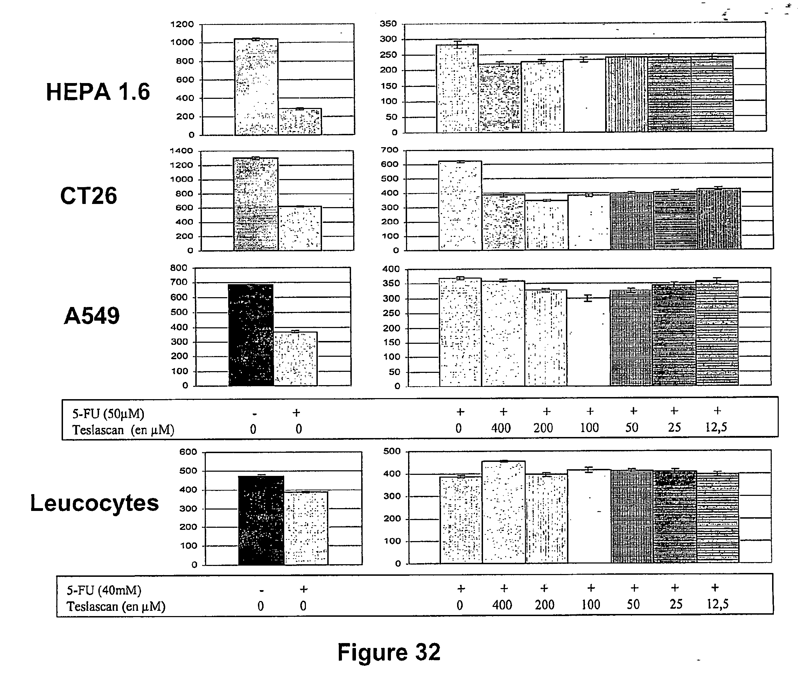

L'addition de MnTBAP (figure 30), de CuDIPS (figure 31) ou de MnDPDP (figure 32) augmente l'activité cytotoxique du 5-FU sur les cellules tumorales. Sur les leucocytes normaux, le MnTBAP n'a qu'une influence très faible sur l'effet cytotoxique du 5-FU (figure 30) ; le CuDIPS (figure 31) augmente cet effet cytotoxique ; en revanche, le MnDPDP (figure 32) l'inhibe.

### EXEMPLE 4 : MODULATION DES EFFETS DES FORMES OXYGENEES REACTIVES SUR L'ADN PAR LA NAC, LE CuDIPS, LE MnTBAP OU LE MnDPDP.

La molécule d'ADN est une des cibles principales de l'effet anti-tumoral des dérivés du platine comme le cisplatine ou l'oxaliplatine. Les dérivés du platine réagissent sur l'ADN en modifiant sa structure tertiaire. Des métalloporphyrines cationiques sont des agents connus pour pouvoir interagir avec l'ADN.

Il a récemment été démontré que des métalloporphyrines ayant des propriétés mimant la SOD pouvaient potentialiser les effets délétères des FRO sur la structure de l'ADN.

Le plasmide pcDNA3.1 (INVITROGEN) purifié a été utilisé pour analyser les altérations potentielles de l'ADN en réponse à l'addition de molécules utilisées dans la chimiothérapies des cancers en présence, ou en l'absence, de modulateurs d'enzymes antioxydantes. Cet ADN a ensuite été stocké à -20°C en 10mM TRIS, 1 mM EDTA jusqu'à son utilisation.

L'ADN plasmidique a été incubé avec de l'oxaliplatine à un rapport molaire de 0,50 dans un volume final de 50µl. Le MnTBAP (5µM), le CuDIPS (5µM), le Mangafodipir (5µM) ou la NAC (5mM) ont alors été ajoutés à la solution. La production d'anion superoxyde a été réalisée par addition, de 200µM xanthine (SIGMA) et 1U de xanthine oxydase (SIGMA). L'incubation a été réalisée dans l'obscurité à 37°C et pendant 24 h. A la fin de la période d'incubation, des aliquots de 10µl ont été soumis à une électrophorèse en gel d'agarose à 0.8% et détectés par coloration au bromure d'ethidium. Les gels ont ensuite été analysés par densitométrie (VILBER LOURMAT, Marnes-la-Vallée, France).

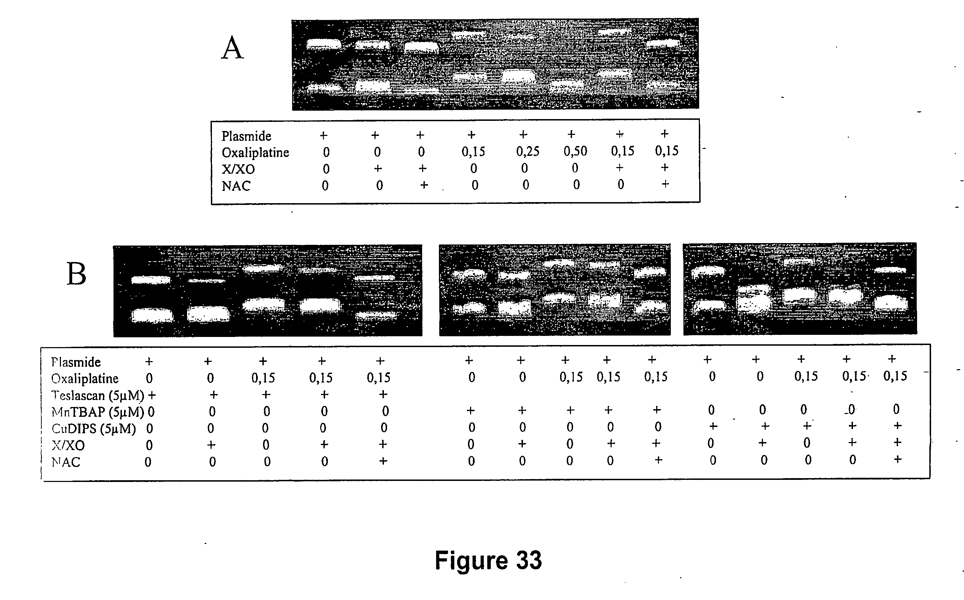

Les résultats sont illustrés dans la figure 33.

Légende de la Figure 33 :
A : Présence (+) ou absence (0) de plasmide ; concentration en oxaliplatine (en µM) ; présence (+) ou absence (0) de xanthine et de xanthine oxydase (X/XO) ; présence (+) ou absence (0) de NAC.
B : Présence (+) ou absence (0) de plasmide ; concentration en oxaliplatine (en µM) ; présence (+) ou absence (0) de xanthine et de xanthine oxydase (X/XO) ; présence (+) ou absence (0) d'anti-oxydant (Teslacan, MnTBAP, CuDIPS ou NAC).

L'incubation d'ADN plasmidique avec de la xanthine et de la xanthine oxydase (X/XO) génère des anions superoxydes qui altèrent la forme native super enroulée du DNA (ADN sous forme I) et favorise la forme circulaire (forme II DNA). Ce phénomène est inhibé par la neutralisation des FRO par la NAC.

L'incubation d'ADN plasmidique avec de l'oxaliplatine induit une altération dose dépendante de la structure de l'ADN maximale au rapport DNA/oxaliplatine de 0.5. Dans ces conditions, on n'observe plus de forme super enroulée et une bande correspondant à la forme III apparaît (forme linéaire). Le rapport forme I/forme II est encore plus abaissé si l'on co-incube l'ADN plasmique avec le système X/XO et de faibles doses d'oxaliplatine. L'incubation avec la NAC diminue les dommages causés à l'ADN.

Dans un second temps, les effets des mimétiques de SOD sur les altérations de l'ADN induites par l'oxaliplatine seul ou associé aux FRO ont été évaluées. L'incubation de l'ADN plasmidique avec du Mangafodipir induit *per se* des dommages à l'ADN comme le montre l'augmentation de la proportion de forme II par rapport au plasmide non traité. Cet effet est amplifié lorsque l'on ajoute soit des anions superoxydes, soit de l'oxaliplatine, et est maximal lorsque Mangafodipir, FRO et oxaliplatine sont co-incubés avec l'ADN plasmidique. Là encore, certains antioxydants comme la NAC inhibent partiellement les altérations de l'ADN.

Un effet similaire est observé lorsque le CuDIPS et, dans une moindre mesure, lorsque le MnTBAP est utilisé comme mimétique de SOD.

### EXEMPLE 7 : EFFETS ANTI-TUMORAUX DE LA NAC, DU CuDIPS, DU MnTBAP ET DU MnDPDP ASSOCIES OU NON AVEC UNE CHIMIOTHERAPIE ANTI-CANCEREUSE CHEZ LA SOURIS.

L'activité anti-tumorale *in vivo* de divers traitements antioxydants a été estimée. Pour ces expériences, des souris femelles BALB/c (pour l'injection de cellules tumorales CT-26) or C57/BL6 (pour l'injection de cellules tumorales Hepa 1-6) âgées de 6 à 8 semaines ont été utilisées. (IFFA CREDO, L'Arbresles, France). Deux millions de cellules tumorales ont été injectées dans le dos des animaux par voie sous-cutanée. Lorsque la taille de la tumeur a atteint 200 à 500 mm3, les animaux ont reçu une injection unique de 20 mg/kg d'oxaliplatine (ELOXATINE^{®}) ou d'une solution saline.

Les souris ont ensuite été traitées par voie intrapéritonéale, deux heures après l'injection d'oxaliplatine ou de solution saline, avec 10 mg/kg de Mangafodipir, de MnTBAP ou de CuDIPS, ou avec 150 mg/kg de NAC, ou avec une solution saline. L'injection des différents antioxydants a été poursuivie pendant un mois (trois injections par semaine aux mêmes doses). Un groupe de souris inoculées avec des cellules tumorales n'a pas été traité.

La taille des tumeurs a été mesurée tous les trois jours. Le volume tumoral a été calculé comme suit: VT (mm3) = (LxW2) /2, ou L est la plus longue et W la plus courte dimension de la tumeur en mm. Quinze souris ont été incluses dans chaque groupe.

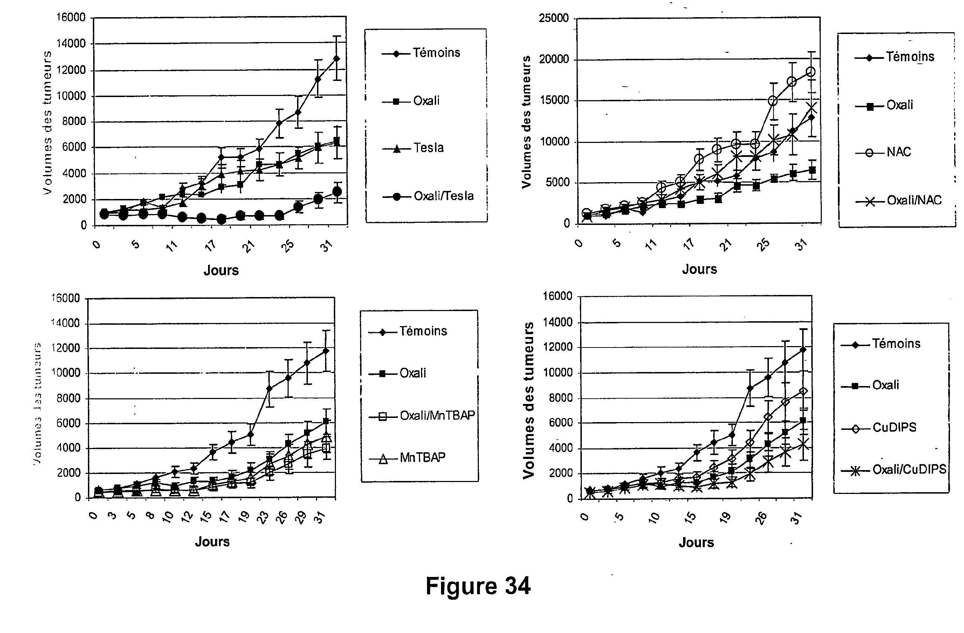

Les résultats de l'expérience basée sur l'injection de cellules tumorales de carcinome colique CT26 à des souris BALB/C sont illustrés dans la Figure 34.

Légende de la Figure 34 :
(◆) témoins,
(■) oxaliplatine,
(▲) teslacan,
(●) oxaliplatine + teslacan,
(○) NAC,
(×) oxaliplatine + NAC,
(Δ) MnTBAP,
(□) oxaliplatine + MnTBAP,
(◊) CUDIPS,
( 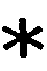 ) oxaliplatine + CUDIPS.

Le volume des tumeurs est indiqué en ordonnée ; en abscisse sont indiqués le nombre de jours suivant l'injection de l'oxaliplatine ou de solution saline.

On observe que l'injection de NAC à des souris non traitées par l'oxaliplatine induit une augmentation de 44% des volumes tumoraux après un mois, par rapport aux souris ne recevant pas de NAC.

Alors que l'administration d'oxaliplatine divise par deux les volumes tumoraux par rapport aux animaux non traités, l'administration de NAC à des souris traitées par oxaliplatine bloque totalement l'effet inhibiteur de l'oxaliplatine sur la croissance tumorale.

Au contraire, l'injection de mimétiques chimiques de SOD comme le MnTBAP, le CuDIPS ou le Mangafodipir diminue respectivement de 59%, 28% et 54% le volume des tumeurs à un mois par rapport aux animaux non traités. En outre, les trois mimétiques de SOD administrés chez des souris traitées à l'oxaliplatine diminue respectivement de 35%, 31% et 63% le volume des tumeurs à un mois par rapport aux animaux traités seulement par l'oxaliplatine.

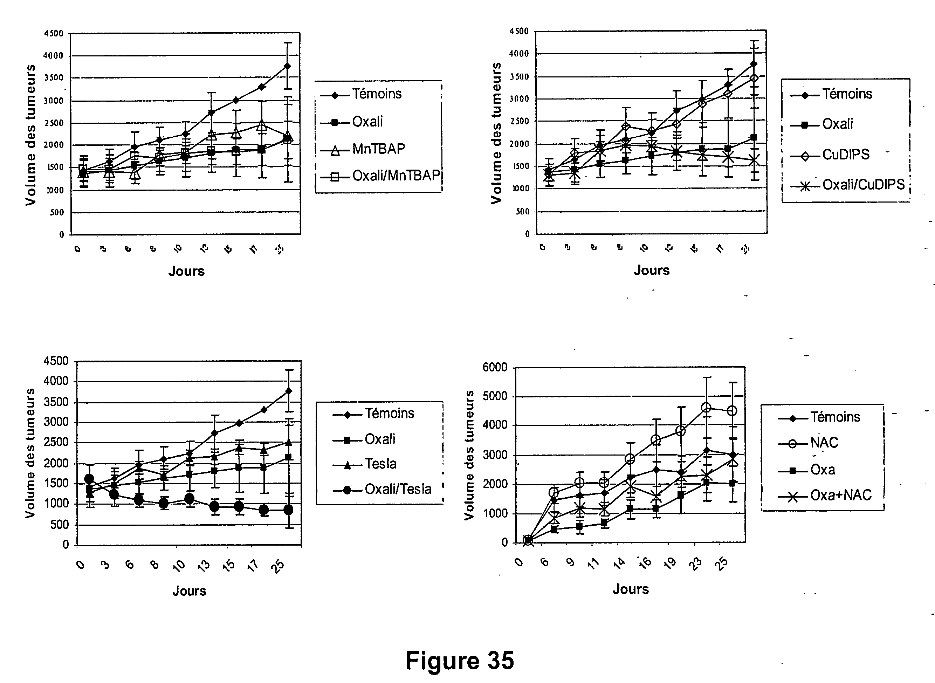

Les résultats de l'expérience basée sur l'injection de cellules Hepa 1-6 à des souris C57BL/6 sont illustrés dans la Figure 35.

Légende de la Figure 35 :
(◆) témoins,
(■) oxaliplatine,
(▲) teslacan,
(●) oxaliplatine + teslacan,
(○) NAC,
(×) oxaliplatine + NAC,
(Δ) MnTBAP,
(□) oxaliplatine + MnTBAP,
(◊) CUDIPS,
( 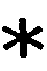 ) oxaliplatine + CUDIPS.

Le volume des tumeurs est indiqué en ordonnée ; en abscisse sont indiqués le nombre de jours suivant l'injection de l'oxaliplatine ou de solution saline.

On observe là encore que l'injection de NAC induit une augmentation de 50% des volumes tumoraux après un mois, par rapport aux souris ne recevant pas de NAC. Alors que l'administration d'oxaliplatine divise par quatre les volumes tumoraux par rapport aux animaux non traités, l'administration de *NAC* à des souris traitées par l'oxaliplatine bloque totalement l'effet inhibiteur de l'oxaliplatine sur la croissance tumorale. Au contraire, l'injection de mimétiques chimiques de SOD comme le MnTBAP, le CuDIPS ou le Mangafodipir diminue de 42%, 9% et 34% respectivement, le volume des tumeurs à un mois par rapport aux animaux non traités. En outre, si la co-administration de MnTBAP et de CuDIPS avec de l'oxaliplatine n'augmente pas de manière significative l'effet anti-tumoral de l'oxaliplatine, l'administration de MnDPDP à des souris traitées à l'oxaliplatine diminue de 63% le volume des tumeurs à un mois par rapport aux animaux traités seulement par l'oxaliplatine (Figure 35).

## Revendications

1. Utilisation du Mangafodipir en tant que principe actif anti-tumoral et protecteur des leucocytes pour l'obtention d'un médicament destiné à un traitement anti-cancéreux.

2. Utilisation selon la revendication 1, **caractérisée en ce que** le Mangafodipir est utilisé en association avec un agent anti-tumoral capable d'induire dans les cellules une production de formes réactives de l'oxygène, choisi parmi la doxorubicine, la mitomycine C, l'étoposide, les dérivés de platine, le tamoxifène, le taxol, le 5-fluoro-uracile, l'irinotécan, la gemcitabine, l'endoxan, la streptozotocine, la bléomycine, la vincristine.

3. Composition pharmaceutique contenant du Mangafodipir, associé avec un agent anti-tumoral choisi parmi la mitomycine C, l'étoposide, les dérivés de platine, le tamoxifène, le 5-fluoro-uracile, l'irinotécan, la gemcitabine, l'endoxan, la streptozotocine, la bléomycine, la vincristine.

## Claims

1. Use of Mangafodipir as an anti-tumour leukocyte-protective active ingredient in order to obtain a medicament intended for anti-cancer treatment.

2. Use according to claim 1, **characterised in that** Mangafodipir is used in association with an anti-tumour agent which is capable of bringing about a production of reactive forms of oxygen in cells and which is selected from doxorubicin, mitomycin C, etoposide, platinum derivatives, tamoxifen, taxol, 5-fluorouracil, irinotecan, gemcitabine, endoxan, streptozotocine, bleomycin, vincristine.

3. Pharmaceutical composition containing Mangafodipir, associated with an anti-tumour agent selected from mitomycin C, etoposide, platinum derivatives, tamoxifen, 5-fluorouracil, irinotecan, gemcitabine, endoxan, streptozotocine, bleomycin, vincristine.

## Patentansprüche

1. Verwendung von Mangafodipir als antitumoraler und leukozytenschützender Wirkstoff zur Herstellung eines Medikaments zur Krebsbehandlung.

2. Verwendung nach Anspruch 1, **dadurch gekennzeichnet, dass** Mangafodipir in Verbindung mit einem Antitumormittel verwendet wird, das in der Lage ist, in den Zellen eine Produktion von reaktiven Formen von Sauerstoff zu induzieren, ausgewählt aus Doxorubicin, Mitomycin C, Etoposid, Platinderivaten, Tamoxifen, Taxol, 5-Fluorouracil, Irinotecan, Gemcitabin, Endoxan, Streptozotocin, Bleomycin und Vincristin.

3. Pharmazeutische Zusammensetzung, enthaltend Mangafodipir in Verbindung mit einem Antitumormittel ausgewählt aus Mitomycin C, Etoposid, Platinderivaten, Tamoxifen, 5-Fluorouracil, Irinotecan, Gemcitabin, Endoxan, Streptozotocin, Bleomycin und Vincristin.
